# EUROPÄISCHE PATENTSCHRIFT

(11) **EP 1 411 844 B1**
(45) Veröffentlichungstag und Bekanntmachung des Hinweises auf die Patenterteilung: **20.11.2013**
(21) Anmeldenummer: 02767188.2
(22) Anmeldetag: 09.07.2002
(51) Int. Cl.: A61B 17/32, A61B 17/28

(54) **MEDIZINISCHES INSTRUMENT MIT ZWEI TEILEN MIT VERBINDUNGSVORRICHTUNG ZU DEREN VERBINDUNG**
MEDICAL INSTRUMENT HAVING TWO PARTS WITH A JOINING DEVICE FOR JOINING THEM
INSTRUMENT MÉDICAL COMPORTANT DEUX PARTIES JOINTES PAR L'INTERMÉDIAIRE D'UN DISPOSITIF DE JONCTION

(30) Priorität: 04.08.2001 DE 10138393
(43) Veröffentlichungstag der Anmeldung: 28.04.2004
(73) Patentinhaber: Aesculap AG, 78532 Tuttlingen (DE)
(72) Erfinder: DWORSCHAK, Manfred, 78589 Dürbheim (DE); LUTZE, Theodor, 78582 Balgheim (DE); MORALES, Pedro, 78532 Tuttlingen-Nendingen (DE); WEISSHAUPT, Dieter, 78194 Immendingen (DE)
(74) Vertreter: Regelmann, Thomas
(86) Internationale Anmeldenummer: PCT/EP2002/007614
(87) Internationale Veröffentlichungsnummer: WO 2003/013375

(56) Entgegenhaltungen:
- US-A- 1 973 569
- US-A- 3 459 187
- US-A- 3 735 763
- US-A- 3 763 726
- US-A- 4 218 821

## Beschreibung

Die Erfindung betrifft ein medizinisches Instrument mit einem ersten Teil und einem zweiten Teil, welche über eine Verbindungsvorrichtung miteinander verbunden sind, wobei das erste Teil eine Haltekammer für das zweite Teil aufweist, welche durch einen ersten Boden und einen gegenüberliegenden zweiten Boden begrenzt ist, und das zweite Teil über mindestens eine Zapfen-Zapfenaufnahme-Verbindung in der Haltekammer an dem ersten Teil sitzt.

Beispielsweise lässt sich eine Klemme dadurch zusammensetzen, dass ein erster Schenkel mit einem zweiten Schenkel verbunden ist.

Aus der US 4,218,821 ist ein Werkzeug bekannt, welches ein Paar von Hebelarmen umfasst, die drehbar miteinander verbunden sind durch einen Drehstift.

Aus der US 3,763,726 ist ein Werkzeug bekannt, welches ein Armpaar umfasst und einen Schachtelverschluss mit einem männlichen Verschlussglied und einem weiblichen Verschlussglied.

Aus der US 1,973,569 ist ein chirurgisches Instrument bekannt, welches zwei relativ zueinander bewegliche Teile umfasst, wobei eines der Teile eine durch dieses durchgehende Öffnung aufweist und die Öffnung zwischen beabstandeten Wänden geformt ist. Das andere Teil erstreckt sich durch diese Öffnung. Die beiden Teile sind schwenkbar aneinander gehalten.

Aus der US 3,459,187 ist ein chirurgisches Instrument bekannt, welches ein erstes Teil und ein zweites Teil aufweist, wobei ein zylindrischer Schwenkstift an dem ersten Teil fixiert ist und durch eine Bohrung des zweiten Teils geht.

Aus der US 3,735,763 ist ein Hämostat bekannt, welcher zwei Griffteile umfasst.

Der Erfindung liegt die Aufgabe zugrunde, ein medizinisches Instrument der eingangs genannten Art zu schaffen, bei welchem sich die beiden Teile auf einfache Weise miteinander verbinden lassen.

Diese Aufgabe wird erfindungsgemäß dadurch gelöst, dass einer Zapfenaufnahme eine Einschubausnehmung zugeordnet ist, von welcher aus ein Zapfen durch Überwindung des Widerstands eines Flankenteils zwischen der Zapfenaufnahme und der Einschubausnehmung in die Zapfenaufnahme schiebbar ist, dass eine Einschubausnehmung an einem Boden ausgebildet und dass eine Zapfenaufnahme in dem ersten Boden und eine Zapfenaufnahme in dem zweiten Boden gebildet ist.

Eine solche Verbindungsvorrichtung lässt sich auf einfache Weise fertigen. Insbesondere lassen sich so Teile aus Kunststoffmaterialien auf einfache Weise miteinander verbinden. Es kann dabei bei der Formung von entsprechenden Kunststoffteilen eine Haltekammer ausgebildet werden oder nachträglich ausgefräst werden. Ebenfalls können entsprechende Zapfenaufnahmen während der Herstellung mit ausgeformt werden oder nachträglich hergestellt werden. Entsprechend lassen sich auch Zapfen herstellen oder nachträglich noch anbringen. Insbesondere läßt sich über eine Zapfen-Zapfenaufnahme-Verbindung ein Drehlager ausbilden, so daß das zweite Teil drehbar zu dem ersten Teil ausgestaltet sein kann.

Es ist aber auch möglich, eine Zapfenaufnahme beispielsweise als Führungsschlitz für den Zapfen auszubilden, so daß das zweite Teil linear verschieblich relativ zu dem ersten Teil ist.

Die beiden Teile lassen sich auf sichere und weitgehend spielfreie Weise miteinander verbinden, wenn das zweite Teil mit dem ersten Teil über Zapfen-Zapfenaufnahme-Verbindungen auf gegenüberliegenden Seiten der Haltekammer verbunden ist. Insbesondere wird dadurch eine große Querstabilität erreicht.

Um eine Drehbarkeit der beiden Teile relativ zueinander zu ermöglichen, sind günstigerweise die Zapfen-Zapfenaufnahme-Verbindungen auf gegenüberliegenden Seiten der Haltekammer fluchtend ausgerichtet und dabei vorzugsweise auch noch rotationssymmetrisch ausgebildet.

Um beispielsweise eine Klemme mit miteinander verschwenkbaren Schenkeln auszubilden ist vorzugsweise das zweite Teil über die Verbindungsvorrichtung drehbar mit dem ersten Teil verbunden.

Ganz besonders günstig ist es, wenn eine Zapfen-Zapfenaufnahme-Verbindung als Drehgelenk ausgebildet ist. Es läßt sich dann auf einfache Weise und insbesondere bei einem medizinischen Instrument aus einem Kunststoffmaterial eine Drehbarkeit von zwei Schenkeln relativ zueinander ausbilden, wobei ein entsprechendes Instrument auf einfache und kostengünstige Weise herstellbar ist; ein solches Instrument läßt sich dann beispielsweise als Einweginstrument einsetzen.

Vorteilhafterweise ist dabei eine Dicke des zweiten Teils an eine Höhe der Haltekammer angepaßt. Über die entsprechende Relation zwischen Dicke des Teils und Höhe der Haltekammer läßt sich beispielsweise die Gängigkeit einer Drehverbindung einstellen: Liegt das zweite Teil an der Haltekammer an, so ist die Verdrehbarkeit des zweiten Teils relativ zum ersten Teil schwerer verglichen mit dem Fall, wenn die Kontaktfläche allein durch die Zapfen-Zapfenaufnahme-Verbindungen gegeben ist.

Bei einer ersten Ausführungsform ist eine Zapfenaufnahme an dem ersten Teil und ein korrespondierender Zapfen an dem zweiten Teil angeordnet. Bei einer damit kombinierbaren oder alternativen Ausführungsform ist eine Zapfenaufnahme an dem zweiten Teil und ein korrespondierender Zapfen an dem ersten Teil angeordnet.

Die Montage des medizinischen Instruments, das heißt die Herstellung der Verbindung, läßt sich auf einfache Weise erreichen, wenn einer Zapfenaufnahme eine Einschubausnehmung zugeordnet ist, von welcher aus ein Zapfen in die Zapfenaufnahme schiebbar ist. Ist entsprechend eine solche Einschubausnehmung abgeschrägt ausgebildet mit abnehmender Öffnungsweite in Richtung Zäpfenaufnahme, dann läßt sich über eine erzeugte Normalkraft der Zapfen in der Art eines Schnappverschlusses in eine Zapfenaufnahme einführen. Darüber hinaus sorgt eine solche Einschubausnehmung für eine Entlastung des Materials insbesondere bei der Montage, da Spannungsspitzen verringert sind.

Günstigerweise ist ein Zapfen in einer Einschubrichtung in eine Zapfenaufnahme abgeschrägt. Der Kraftaufwand, um über eine abgeschrägte Einschubausnehmung den Zapfen in eine Zapfenaufnahme zu bringen ist dann erheblich geringer, als den Zapfen wieder aus der Zapfenaufnahme herauszuführen. Dadurch läßt sich eine sichere Verbindung herstellen.

Die erfindungsgemäße Verbindungsvorrichtung läßt sich auf vorteilhafte Weise einsetzen, wenn das erste Teil aus Kunststoffmaterial und/oder das zweite Teil ebenfalls aus einem Kunststoffmaterial gefertigt ist. Wie oben bereits erwähnt lassen sich die entsprechenden Zapfen und Zapfenaufnahmen auf einfache Weise während der Formung der Haltekammer und des zweiten Teils herstellen.

Es kann vorgesehen sein, daß eine Zapfenaufnahme gegenüber dem Außenraum geschlossen ist (geschlossene Zapfenaufnahme), indem die Zapfenaufnahme nicht als Durchbrechung ausgebildet ist, sondern zwischen dieser und dem Außenraum noch eine Trennwand liegt. Auf diese Weise läßt sich beispielsweise ein Drehlager gegenüber Verschmützungen und Beschädigungen schützen.

Es kann alternativ dazu vorgesehen sein, daß eine Zapfenaufnahme gegenüber dem Außenraum offen ist (offene Zapfenaufnahme). Eine derartige Zapfenaufnahme lässt sich auf einfache Weise herstellen. Insbesondere lässt sich auch über nachträgliche Materialbearbeitung wie Fräsbearbeitung oder Bohrung herstellen. Darüber hinaus lassen sich gegenüberliegende, miteinander fluchtend ausgebildete Zapfenaufnahmen auf einfache Weise als offene Zapfenaufnahmen herstellen.

Die nachfolgende Beschreibung bevorzugter Ausführungsformen dient im Zusammenhang mit der Zeichnung der näheren Erläuterung der Erfindung. Es zeigen:
- Figur 1: eine schematische Ansicht einer Klemme, welche mit einer erfindungsgemäßen Verbindungsvorrichtung versehen ist;
- Figur 2: eine Teilansicht der Klemme gemäß Figur 1;
- Figur 3: eine Schnittansicht längs der Linie 3-3 gemäß Figur 2 einer ersten Ausführungsform einer erfindungsgemäßen Verbindungsvorrichtung;
- Figur 4: eine Schnittansicht längs der Linie 3-3 gemäß Figur 2 einer Ausführungsform einer Verbindungsvorrichtung, welche nicht unter die Ansprüche fällt;
- Figur 5: eine Schnittansicht längs der Linie 5-5 gemäß Figur 2 während der Montage eines zweiten Teils an einem ersten Teil;
- Figur 6: die gleiche Ansicht wie Figur 5, wobei jetzt das zweite Teil montiert ist;
- Figur 7: eine Variante einer Haltekammer zur Aufnahme des zweiten Teils und
- Figur 8: eine Teilansicht eines weiteren Instruments (eines endoskopischen Instruments) bei dem ein erstes Teil und ein zweites Teil über eine erfindungsgemäße Verbindungsvorrichtung verbunden sind.

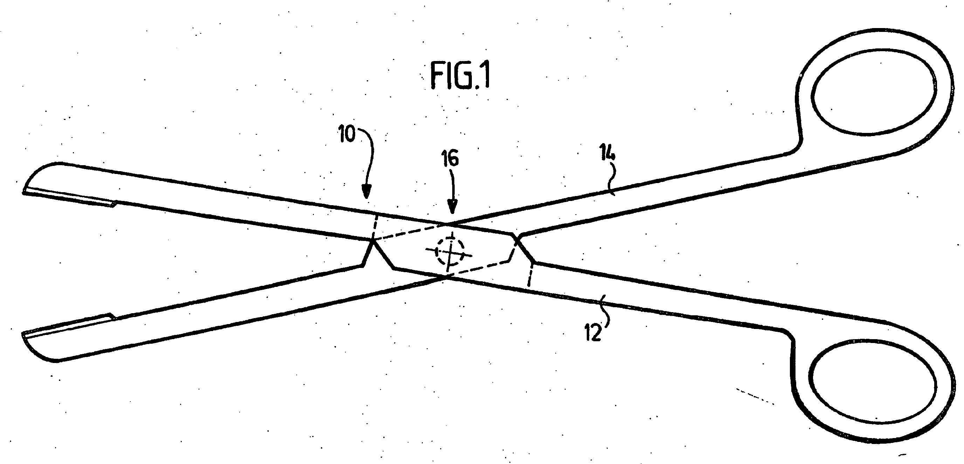

In Figur 1 ist als Ausführungsbeispiel eines erfindungsgemäßen medizinischen Instruments eine Klemme 10 gezeigt. Diese umfaßt als erstes Teil 12 einen ersten Schenkel und als zweites Teil 14 einen zweiten Schenkel. Über eine Verbindungsvorrichtung 16 sind die beiden Schenkel 12, 14 drehbar miteinander verbunden.

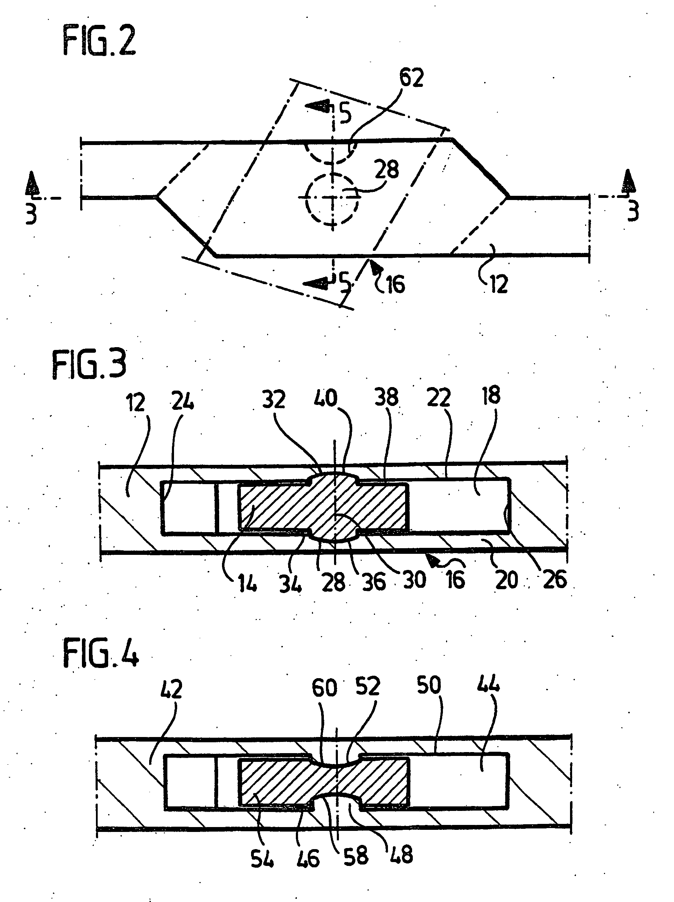

Es ist dabei, wie in den Figuren 3 bis 7 gezeigt, in dem ersten Teil 12 eine Haltekammer 18 angeordnet, welche das zweite Teil 14 mindestens teilweise aufnimmt und über die das zweite Teil 14 mit dem ersten Teil 12 verbunden ist. Es kann dabei vorgesehen sein, daß das zweite Teil 14 relativ zu dem ersten Teil 12 beweglich und insbesondere drehbar ist, so daß die Haltekammer 18 genügend groß ausgebildet sein muß, daß ein Bewegungsspielraum für das zweite Teil 14 vorliegt. Außerdem sollte die Haltekammer 18 genügend groß ausgebildet sein, daß ihre Begrenzungswände federn können, um, wie weiter unten näher beschrieben, Zapfen-Zapfenaufnahme-Verbindungen herzustellen.

Die Haltekammer 18 ist durch eine Ausnehmung und insbesondere Durchbrechung in dem ersten Teil 12 gebildet. Sie wird durch einen ersten Boden 20 und einen gegenüberliegenden zweiten Boden 22 begrenzt. Quer zu dem ersten Boden 20 und dem zweiten Boden 22 begrenzt eine erste Wand 24 und eine gegenüberliegende zweite Wand 26 die Haltekammer 18. Es kann dabei vorgesehen sein, daß die erste Wand 24 und/oder die zweite Wand 26 abgerundet ausgebildet sind.

Die gegenüberliegenden Böden 20 und 22 sind im wesentlichen parallel zueinander und eben ausgebildet wie auch die Wände 24 und 26.

In dem ersten Boden 20 ist rotationssymmetrisch um eine Achse 30 eine Zapfenaufnahme 28 durch eine entsprechende Ausnehmung gebildet. Gezeigt ist dabei eine geschlossene Zapfenaufnahme, die zum Außenraum hin mit einer Begrenzungswand versehen ist. Sie kann aber auch zum Außenraum hin offen sein.

In dem zweiten Boden 22 ist ebenfalls rotationssymmetrisch um die Achse 30 eine Zapfenaufnahme 32 gebildet.

Das erste Teil 12 ist an seiner dem Boden 20 zugewandten Unterseite 34 mit einem Zapfen 36 versehen, welcher in die Zapfenaufnahme 28 eintaucht. Ferner ist das zweite Teil 14 an seiner dem zweiten Boden 22 zugewandten Oberseite 38 mit einem fluchtend zum Zapfen 36 ausgerichteten Zapfen 40 versehen, welcher in die Zapfenaufnahme 32 eintaucht. Der Abstand längs der Achse 30 zwischen einer Begrenzungswand der Zapfenaufnahme 28 und einer Begrenzungswand der Zapfenaufnahme 32 ist dabei größer als der Abstand der Böden 20 und 22.

Auf diese Weise wird das zweite Teil über die Zapfen-Zapfenaufnahme-Verbindung 28, 36 und über die Zapfen-Zapfenaufnahme-Verbindung 32, 40 drehbar an dem ersten Teil 12 gehalten. Die Verbindungsvorrichtung selber bildet dabei ein Drehgelenk aus, das heißt, die Zapfen 36 und 40 wirken als Drehwellen.

Es kann aber auch vorgesehen sein, daß das zweite Teil 14 verschieblich an dem ersten Teil 12 angeordnet ist, wenn entsprechend die zugeordneten Zapfenaufnahmen als Führungsschlitz für die Zapfen ausgebildet sind, so daß die Zapfen längs dieser Führungsschlitze beweglich sind und damit das zweite Teil 14 längsverschieblich zum ersten Teil 12 ist.

Es kann auch vorgesehen sein, daß die beiden Teile 14 und 12 unbeweglich miteinander verbunden sind, indem beispielsweise die Zapfen und Zapfenaufnahmen auf gegenüberliegenden Seiten nicht fluchtend aufeinander ausgerichtet sind.

Bei einer zweiten Ausführungsform, welche in Figur 4 gezeigt ist, ist im Vergleich zu der ersten Ausführungsform gemäß Figur 3 die Ausbildung der Zapfen und Zapfenaufnahmen umgekehrt: In einem ersten Teil 42 ist wiederum eine Haltekammer 44 gebildet. Ein erster Boden 46 dieser Haltekammer 44 ist dabei mit einem Zapfen 48 versehen, welcher in Richtung eines zweiten gegenüberliegenden Bodens 50 der Haltekammer 44 weist. Dieser ist ebenfalls mit einem Zapfen 52 versehen, welcher insbesondere fluchtend zu dem Zapfen 48 ausgerichtet ist. Zur Ausbildung einer Drehgelenk-Verbindung eines zweiten Teils 54 mit dem ersten Teil 42 sind dabei die beiden Zapfen 48 und 52 rotationssymmetrisch um eine Verbindungsachse 56 zwischen den Zapfen 48 und 52.

In dem zweiten Teil 54 ist auf einer dem ersten Boden 46 zugewandten Seite eine Ausnehmung 58 als Zapfenaufnahme gebildet. Ferner ist auf der gegenüberliegenden Seiten dem zweiten Boden 50 zugewandt in dem zweiten Teil 54 eine Zapfenaufnahme 60 für den Zapfen 52 gebildet.

Bei verbundenem ersten Teil 42 und zweiten Teil 54 taucht der Zapfen 52 in die Zapfenaufnahme 60 ein und der Zapfen 48 in die Zapfenaufnahme 56. Auf diese Weise sitzt das zweite Teil 54 an dem ersten Teil 42 und das zweite Teil 54 ist relativ zum ersten Teil 42 drehbar.

Zur Erleichterung der Herstellung der Verbindung zwischen den beiden Teilen 12 und 14 ist in dem ersten Teil 12 jeder Zapfenaufnahme eine Einschubausnehmung 62 zugeordnet (Figur 2, Figuren 5 bis 7). Eine solche Einschubausnehmung 62 ist in einer Montagerichtung 64, in der das zweite Teil 14 zur Herstellung der Verbindung in die Haltekammer 18 eingeschoben wird, abgeschrägt. Dies bedeutet, daß eine Öffnungsweite der Einschuböffnung 62 in Montagerichtung 64 abnimmt.

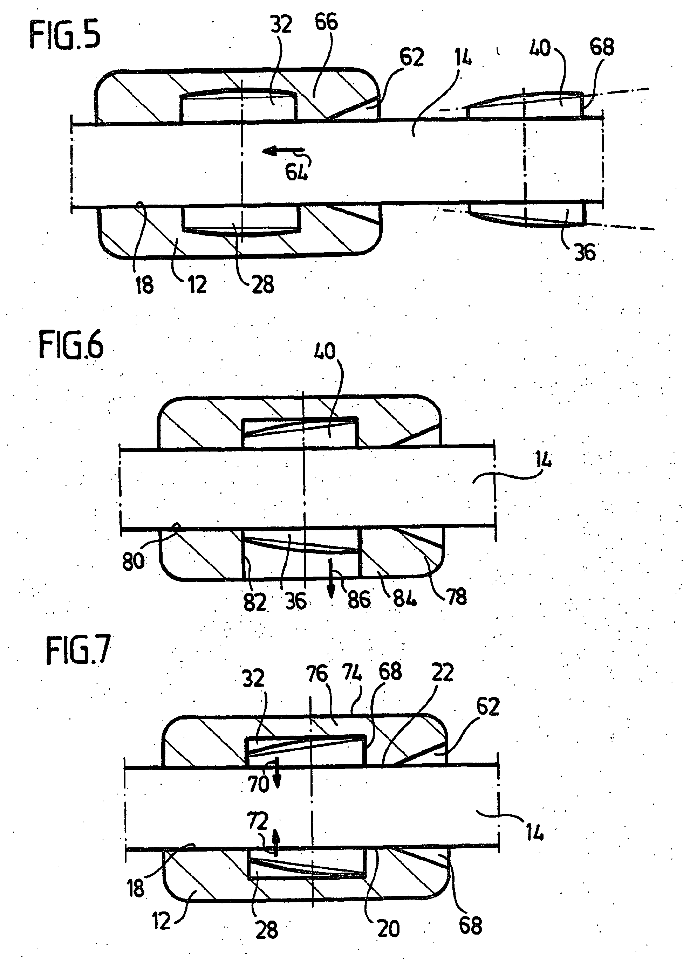

Ferner sind die jeweiligen Zapfen 36 und 40 bezogen auf die Montagerichtung 64 abgeschrägt. Die Einschubausnehmung 62 für den zugeordneten Zapfen, beispielsweise 40, dient dann als Montagehilfe: Der Zapfen 40 mit seiner Abschrägung wird beim Einschieben des zweiten Teils 14 in die Haltekammer 18 in die Einschubausnehmung 62 eingeschoben. Durch weitere Kraftbeaufschlagung lässt sich der Widerstand eines Flankenteils 66 zwischen beispielsweise dem Zapfen 32 und der Einschuböffnung 62 bei entsprechender Materialverwendung überwinden, wobei die Abschrägung des Zapfens 32 eine Normalkraft auf diesen Flankenteil 66 ausübt, um diesen während der Kraftausübung nach oben, von dem zweiten Teil 14 weg, zu drücken. Taucht dann der Zapfen 40 in die Zapfenaufnahme 32 ein, dann ist er dort in der Art eines Schnappverschlusses gehalten, da ein Kraftaufwand zum Wiederherausziehen erforderlich ist. Durch die Abschrägung des Zapfens 40 ist der Montagerichtung 64 abgewandt eine steile Flanke 68 und insbesondere im Wesentlichen senkrecht abstehende Flanke gebildet. Liegt diese steile Flanke 68 in der Zapfenaufnahme 32 an (Figur 6), dann ist das Herausziehen des zweiten Teils 14 entgegen der Montagerichtung 12 gesperrt und es ist ein sehr hoher Kraftaufwand erforderlich, um das zweite Teil wieder herauszuziehen; in der Regel ist dieser Kraftaufwand höher als derjenige, welcher benötigt wird, um das zweite Teil 14 mit dem ersten Teil 12 über die Zapfen-Zapfenaufnahme-Verbindungen 32, 40 und 28, 36 zu verbinden.

Das erste Teil 12 mit seiner Haltekammer 18, seinen Zapfenaufnahmen 32 und 28 und seinen Einschubausnehmungen 62 lässt sich vorteilhafterweise aus einem Kunststoffmaterial herstellen, beispielsweise mittels eines Spritzgußverfahrens. Die Einschuböffnungen 62, 68 (Figur 6) an den gegenüberliegenden Böden 22, 20 der Haltekammer 18 lassen sich mittels eines Schiebers ausformen, welcher nach der Formung des ersten Teils 12 herausgezogen wird. Dieser Schieber hat eine entsprechende Gestalt, um eben die Einschuböffnungen 62 und 68 ausbilden zu können.

Ebenso sind zur Bildung der Zapfenaufnahmen 28 und 32 entsprechende Schieber vorgesehen, die nach der Beendigung beispielsweise des Spritzgusses aus den entsprechenden Zapfenaufnahmen 28, 32 herausgezogen werden. Dies ist in Figur 6 durch die Pfeile 70 und 72 angedeutet.

Bei dem in den Figuren 3, 4 und 5, 6 gezeigten Ausführungsbeispielen sind die Zapfenaufnahmen jeweils durch eine nicht-durchgehende Ausnehmung (geschlossene Zapfenaufnahme) gebildet, das heißt zwischen einer Oberseite 74 beispielsweise des ersten Teils 12 und der Zapfenaufnahme 32 liegt noch eine Trennwand 76 (Figur 6), welche die Zapfenaufnahme 32 gegenüber dieser Oberseite 74 verschließt.

Bei einem Ausführungsbeispiel, welches in Figur 7 gezeigt ist, ist ein erstes Teil 78 vorgesehen, welches wiederum eine Haltekammer 80 aufweist. Während der Herstellung wird von einer Unterseite 84 her in dem ersten Teil 12 eine sacklochartige Ausnehmung gebildet, indem beispielsweise ein entsprechender Schieber in der Form fixiert ist und nach beispielsweise einem Spritzverfahren dieser Schieber in einer Richtung 86 herausgezogen wird. Auf diese Weise lassen sich in einem Vorgang die Zapfenaufnahmen für den Zapfen 40 und den Zapfen 36 gleichzeitig bilden, wobei insbesondere das Herausziehen des Schiebers zur Bildung der Ausnehmung 82 (offene Zapfenaufnahme) erleichtert ist, da nur ein einziger Schieber vorgesehen werden muß. Der Schieber kann auch direkt von der Unterseite 84 her entnommen werden und muß nicht, im Gegensatz zu dem Ausführungsbeispiel gemäß Figur 6, von der Haltekammer 18 aus entnommen werden.

Es können auch zwei gegenüberliegende offene Zapfenausnehmungen vorgesehen sein. Eine offene Zapfenausnehmung läßt sich auch über einen Fräsvorgang oder Bohrvorgang von einer Außenseite eines Teils her herstellen.

Ansonsten funktioniert die Verbindungsvorrichtung gemäß Figur 7 so wie oben beschrieben.

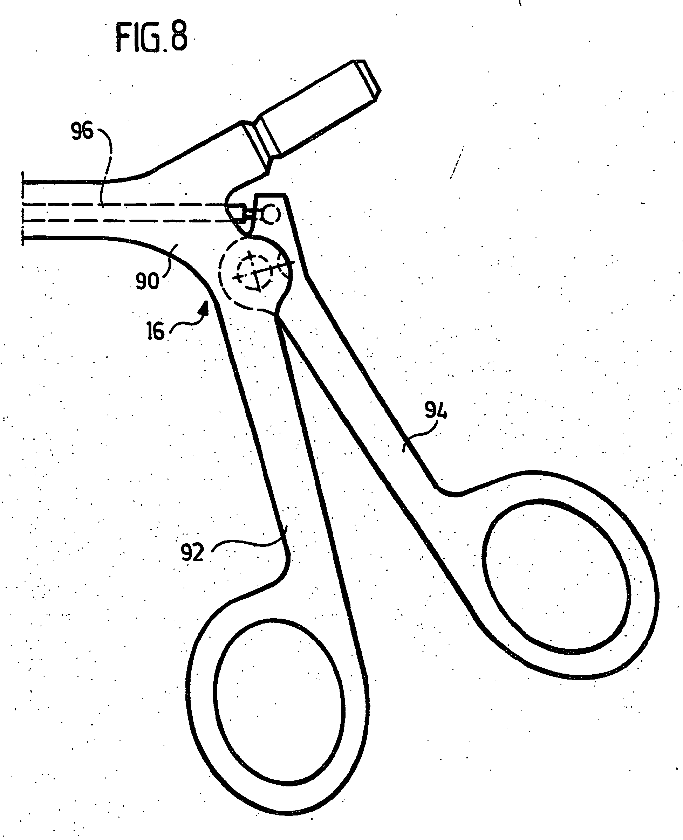

Bei einem weiteren Ausführungsbeispiel eines medizinischen Instruments, insbesondere eines endoskopischen Instruments, welches in Figur 8 gezeigt ist, ist ein erstes Teil 90 mit einem einstückig daran sitzenden Griffelement 92 über eine Verbindungsvorrichtung 16 mit einem zweiten Teil 94, welches ebenfalls als Griffelement ausgebildet ist, verbunden. Die beiden Griffelemente 92 und 94 sind dabei relativ zueinander schwenkbar, wobei das Schwenklager mittels der Verbindungsvorrichtung 16 wie oben beschrieben gebildet ist. Durch Verschwenkung des Griffelements 94 gegenüber dem Griffelement 92 läßt sich dabei ein Betätigungselement 96 in dessen Längsrichtung linear verschieben.

## Patentansprüche

1. Medizinisches Instrument mit einem ersten Teil (12) und einem zweiten Teil (14), welche über eine Verbindungsvorrichtung (16) miteinander verbunden sind, wobei das erste Teil (12) eine Haltekammer (18) für das zweite Teil (14) aufweist, welche durch einen ersten Boden (20; 46) und einen gegenüberliegenden zweiten Boden (22; 50) begrenzt ist, und das zweite Teil (14) über mindestens eine Zapfen-Zapfenaufnahme-Verbindung (28, 36, 32, 40) in der Haltekammer (18) an dem ersten Teil (12) sitzt, **dadurch gekennzeichnet, dass** eine Einschubausnehmung (62; 68) an dem jeweiligen Boden (20; 22) ausgebildet und dass eine Zapfenaufnahme (28) in dem ersten Boden (20) und eine Zapfenaufnahme (32) in dem zweiten Boden (22) gebildet ist, dass korrespondierende Zapfen (40) an dem zweiten Teil (14) angeordnet sind, und dass einer Zapfenaufnahme (28; 32) eine Einschubausnehmung (62; 68) zugeordnet ist, von welcher aus der jeweilige Zapfen (40) durch Überwindung des Widerstands eines Flankenteils (66) zwischen der Zapfenaufnahme (28; 32) und der Einschubausnehmung (62; 68) in die Zapfenaufnahme (28; 32) schiebbar ist.

2. Medizinisches Instrument nach Anspruch 1, **dadurch gekennzeichnet, dass** das zweite Teil (14) mit dem ersten Teil (12) über Zapfen-Zapfenaufnahme-Verbindungen (28, 36, 32, 40) auf gegenüberliegenden Seiten (20, 22) der Haltekammer (18) verbunden ist.

3. Medizinisches Instrument nach Anspruch 2, **dadurch gekennzeichnet, dass** die Zapfen-Zapfenaufnahme-Verbindungen (28, 36, 32, 40) auf gegenüberliegenden Seiten (20; 22) der Haltekammer (18) fluchtend ausgebildet sind.

4. Medizinisches Instrument nach einem der vorangehenden Ansprüche, **dadurch gekennzeichnet, dass** das zweite Teil (14) über die Verbindungsvorrichtung (16) drehbar mit dem ersten Teil (12) verbunden ist.

5. Medizinisches Instrument nach einem der vorangehenden Ansprüche, **dadurch gekennzeichnet, dass** eine Zapfen-Zapfenaufnahme-Verbindung (28, 36) als Drehgelenk ausgebildet ist.

6. Medizinisches Instrument nach einem der vorangehenden Ansprüche, **dadurch gekennzeichnet, dass** eine Dicke des zweiten Teils (14) an eine Höhe der Haltekammer (18) angepasst ist.

7. Medizinisches Instrument nach einem der vorangehenden Ansprüche, **dadurch gekennzeichnet, dass** eine Zapfenaufnahme (28) an dem ersten Teil (12) angeordnet ist und ein korrespondierender Zapfen (36; 40) an dem zweiten Teil (14) angeordnet ist.

8. Medizinisches Instrument nach einem der vorangehenden Ansprüche, **dadurch gekennzeichnet, dass** eine Einschubausnehmung (62; 68) abgeschrägt ausgebildet ist mit abnehmender Öffnungsweite in Richtung der Zapfenaufnahme (28; 32).

9. Medizinisches Instrument nach einem der vorangehenden Ansprüche, **dadurch gekennzeichnet, dass** ein Zapfen (40) in einer Einschubrichtung (64) in eine Zapfenaufnahme (32) abgeschrägt ist.

10. Medizinisches Instrument nach einem der vorangehenden Ansprüche, **dadurch gekennzeichnet, dass** das erste Teil (12) aus einem Kunststoffmaterial gefertigt ist.

11. Medizinisches Instrument nach einem der vorangehenden Ansprüche, **dadurch gekennzeichnet, dass** das zweite Teil (14) aus einem Kunststoffmaterial gefertigt ist.

12. Medizinisches Instrument nach einem der vorangehenden Ansprüche, **dadurch gekennzeichnet, dass** eine Zapfenaufnahme (28; 32) gegenüber dem Außenraum geschlossen ist.

13. Medizinisches Instrument nach einem der vorangehenden Ansprüche, **dadurch gekennzeichnet, dass** eine Zapfenaufnahme (82) gegenüber dem Außenraum offen ist.

## Claims

1. Medical instrument having a first part (12) and a second part (14) which are connected together by a connecting device (16), wherein the first part (12) comprises a retaining chamber (18) for the second part (14), which is bounded by a first base (20; 46) and an oppositely located second base (22; 50), and the second part (14) is seated on the first part (12) by means of at least one spigot and spigot-seating connecting arrangement (28, 36, 32, 40) in the retaining chamber (18), **characterized in that** an insertion recess (62; 68) is formed on the respective base (20; 22), and **in that** a spigot seating (28) is formed in the first base (20) and a spigot seating (32) in the second base (22), **in that** corresponding spigots (40) are arranged on the second part (14), and **in that** associated with a spigot seating (28; 32) is an insertion recess (62; 68) from which the respective spigot (40) can be pushed into the spigot seating (28; 32) by overcoming the resistance of a flank part (66) between the spigot seating (28; 32) and the insertion recess (62; 68).

2. Medical instrument in accordance with claim 1, **characterized in that** the second part (14) is connected to the first part (12) by means of spigot and spigot-seating connecting arrangements (28, 36, 32, 40) on opposite sides (20, 22) of the retaining chamber (18).

3. Medical instrument in accordance with claim 2, **characterized in that** the spigot and spigot-seating connecting arrangements (28, 36, 32, 40) on opposite sides (20; 22) of the retaining chamber (18) are in alignment.

4. Medical instrument in accordance with any one of the preceding claims, **characterized in that** the second part (14) is connected to the first part (12) via the connecting device (16) in rotatable manner.

5. Medical instrument in accordance with any one of the preceding claims, **characterized in that** a spigot and spigot-seating connecting arrangement (28, 36) is in the form of a swivel joint.

6. Medical instrument in accordance with any one of the preceding claims, **characterized in that** a thickness of the second part (14) is adapted to a height of the retaining chamber (18).

7. Medical instrument in accordance with any one of the preceding claims, **characterized in that** a spigot seating (28) is arranged on the first part (12) and a corresponding spigot (36; 40) is arranged on the second part (14).

8. Medical instrument in accordance with any one of the preceding claims, **characterized in that** an insertion recess (62; 68) is tapered with a decreasing aperture width in the direction of the spigot seating (28; 32).

9. Medical instrument in accordance with any one of the preceding claims, **characterized in that** a spigot (40) is tapered in a direction of insertion (64) into a spigot seating (32).

10. Medical instrument in accordance with any one of the preceding claims, **characterized in that** the first part (12) is manufactured from a synthetic material.

11. Medical instrument in accordance with any one of the preceding claims, **characterized in that** the second part (14) is manufactured from a synthetic material.

12. Medical instrument in accordance with any one of the preceding claims, **characterized in that** a spigot seating (28; 32) is closed with respect to the exterior.

13. Medical instrument in accordance with any one of the preceding claims, **characterized in that** a spigot seating (82) is open with respect to the exterior.

## Revendications

1. Instrument médical comprenant une première partie (12) et une deuxième partie (14), qui sont reliées mutuellement par l'intermédiaire d'un dispositif de liaison (16), la première partie (12) présentant, pour la deuxième partie (14), un logement de maintien (18) qui est délimité par un premier fond de base (20; 46) et un deuxième fond de base (22; 50) opposé, et la deuxième partie (14) étant logée, par l'intermédiaire d'au moins une liaison tourillon - alvéole de tourillon (28, 36, 32, 40), dans le logement de maintien (18) sur la première partie (12),
**caractérisé en ce qu'**un évidement d'insertion (62; 68) est formé sur le fond de base respectif (20; 22) et qu'une alvéole de tourillon (28) est formée dans le premier fond de base (20) et une alvéole de tourillon (32) dans le deuxième fond de base (22), **en ce que** des tourillons correspondants (40) sont agencés sur la deuxième partie, et **en ce qu'**à une alvéole de tourillon (28; 32) est associé un évidement d'insertion (62; 68) à partir duquel le tourillon (40) respectif peut être inséré par coulissement dans l'alvéole de tourillon (28; 32), en surmontant la résistance d'une partie à profil de flanc (66) entre l'alvéole de tourillon (28; 32) et l'évidement d'insertion (62; 68).

2. Instrument médical selon la revendication 1, **caractérisé en ce que** la deuxième partie (14) est reliée à la première partie (12) par l'intermédiaire de liaisons tourillon - alvéole de tourillon (28, 36, 32, 40) sur des côtés opposés (20, 22) du logement de maintien (18).

3. Instrument médical selon la revendication 2, **caractérisé en ce que** les liaisons tourillon - alvéole de tourillon (28, 36, 32, 40) sont réalisées de manière mutuellement alignées, sur des côtés opposés (20; 22) du logement de maintien (18).

4. Instrument médical selon l'une des revendications précédentes, **caractérisé en ce que** la deuxième partie (14) est reliée de manière rotative à la première partie (12) par l'intermédiaire du dispositif de liaison (16).

5. Instrument médical selon l'une des revendications précédentes, **caractérisé en ce qu'**une liaison tourillon - alvéole de tourillon (28, 36) est réalisée en tant qu'articulation de rotation.

6. Instrument médical selon l'une des revendications précédentes, **caractérisé en ce qu'**une épaisseur de la deuxième partie (14) est adaptée à une hauteur du logement de maintien (18).

7. Instrument médical selon l'une des revendications précédentes, **caractérisé en ce qu'**une alvéole de tourillon (28) est agencée sur la première partie (12) et un tourillon correspondant (36; 40) sur la deuxième partie (14).

8. Instrument médical selon l'une des revendications précédentes, **caractérisé en ce qu'**un évidement d'insertion (62; 68) est d'une configuration biseautée, avec une largeur d'ouverture décroissante en direction de l'alvéole de tourillon (28; 32).

9. Instrument médical selon l'une des revendications précédentes, **caractérisé en ce qu'**un tourillon (40) est biseauté dans une direction d'insertion (64) dans une alvéole de tourillon (32).

10. Instrument médical selon l'une des revendications précédentes, **caractérisé en ce que** la première partie (12) est fabriquée en une matière plastique.

11. Instrument médical selon l'une des revendications précédentes, **caractérisé en ce que** la deuxième partie (14) est fabriquée en une matière plastique.

12. Instrument médical selon l'une des revendications précédentes, **caractérisé en ce qu'**une alvéole de tourillon (28; 32) est fermée par rapport à l'espace extérieur.

13. Instrument médical selon l'une des revendications précédentes, **caractérisé en ce qu'**une alvéole de tourillon (82) est ouverte par rapport à l'espace extérieur.
